# EUROPEAN PATENT SPECIFICATION

(11) **EP 2 124 553 B1**
(45) Date of publication and mention of the grant of the patent: **01.03.2017**
(21) Application number: 08744246.3
(22) Date of filing: 24.03.2008
(51) Int. Cl.: A61K 31/513, A61K 31/7068, A61P 19/02

(54) **METHOD FOR TREATING AND PREVENTING ARTHRITIS**
VERFAHREN ZUR BEHANDLUNG UND PROPHYLAXE VON ARTHRITIS
PROCÉDÉ DE TRAITEMENT ET DE PRÉVENTION DE L'ARTHRITE

(30) Priority: 23.03.2007 US 896549 P
(43) Date of publication of application: 02.12.2009
(73) Proprietor: Southern Research Institute, Birmingham, Alabama 35205-5305 (US)
(72) Inventor: SECRIST, John, A., Birmingham, AL 35243 (US); WAUD, William, R., Mountain Brook, AL 35223 (US); QU, Zhican, Birmingham, AL 35226 (US); CUI, Xiangmin, San Bruno, CA 94066 (US)
(74) Representative: Zanoli, Enrico
(86) International application number: PCT/US2008/058008
(87) International publication number: WO 2008/118852

(56) References cited:
- US-A1- 2003 087 873
- US-B1- 6 576 621
- BAYLISS G E ET AL: "Cytarabine therapy for rheumatoid arthritis.", CLINICAL AND EXPERIMENTAL RHEUMATOLOGY 1992 JUL-AUG LNKD- PUBMED:1395227, vol. 10, no. 4, July 1992 (1992-07), pages 420-421, XP009142628, ISSN: 0392-856X
- MCCUNE W J ET AL: "Immunosuppressive drug therapy for rheumatic disease", CURRENT OPINION IN RHEUMATOLOGY, CURRENT SCIENCE, LONDON, GB, vol. 5, no. 3, 1 May 1993 (1993-05-01), pages 282-292, XP009142660, ISSN: 1040-8711

## Description

### Technical Field

The present disclosure relates to treating or preventing arthritis including rheumatoid arthritis in a patient in need thereof by administering to the patient certain thioarabinofuranosyl compounds. Compounds employed according to the present disclosure have exhibited good anti-arthritic activity as well as demonstrating a prophylactic effect for preventing or at least substantially preventing arthritis. Compounds employed according to the present disclosure are in the beta configuration as contrasted to the alpha configuration.

### Background

Despite the development of many arthritis drugs, arthritis continues to be a world wide serious disease due to an increasing aging population. Even though the death rate due to arthritis is low, the quality of life of an individual who suffers from this disease is sacrificed with lowered activity level and productivity.

Among many types of arthritis, the most significant one is rheumatoid arthritis. Rheumatoid arthritis is an autoimmune disease by the action of auto-reactive T lymphocytes. T lymphocytes cause rheumatoid anthritis via delayed type hypersensitivity. It is not fully understood which antigen is recognized by T lymphocytes to cause this disease. Type II collagen is known to be the most probable one, but other possibilities cannot be excluded. Anti-histone autoantibody has been discovered even though it is not clear that this antibody is the cause of the disease.

Many drugs have been used to treat rheumatoid arthritis without a complete relief of the symptoms. Conventional drugs include non-steroidal anti-inflammatory drugs (NSAIDs, aspirin, ibuprofen), gold salt, penicillamine, and steroidal hormones. The steroidal hormones, which are the most potent and effective, have side effects when taken for a long period. Recently, recombinant soluble receptor of tumor necrosis factor (TNF), that plays a major role in the inflammation mechanism, is on trial as a new treatment of rheumatoid arthritis. However, an improved formulation to treat symptoms of rheumatoid arthritis such as inflammation is desired.

Collagen-induced arthritis (CIA) has been used as an animal model of the T-lymphoidal rheumatoid arthritis (Autoimmunity to Type II collagen: Experimental model of arthritis, J. Exp. Med. 146; 857-868 (1977)). When type II collagen was injected into mice, which are prone to develop arthritis, arthritis was induced within 2 weeks with symptoms such as formation of pannus, erosion of cartilage and bone. Like rheumatoid arthritis, CIA also has the humoral and the cellular immune responses against collagen.

### Summary

The present invention relates to 1-(4-thio-[beta]-D-arabinofuranosyl) cytosine for use in a method for treating or preventing rheumatoid arthritis which comprises administering to a host in need thereof an amount of compound effective for treating or preventing rheumatoid arthritis.

Brief Description of Drawings
Figure 1 illustrates mean mouse weights over the trial period.
Figure 2 shows the incidence of a skin rash in one of the mice in the 20mg/kg/day group.
Figure 3 shows splenomegaly in all mice in the high dose (60mg/kg/day) group.
Figure 4 shows the incidence of arthritis in mice treated with the test compound according to this disclosure at various doses.
Figure 5 shows the mean onset of collagen-induced arthritis in the various treatment groups.
Figure 6 shows the incidence of arthritis over time.
Figure 7 shows an analysis of the cumulative number of arthritic paws in treated and control animals.
Figure 8 shows the analysis of mean disease severity.
Figure 9 shows the levels of anti-CII antibody in sera taken prior to the experiment, 14 days after immunization, onset of arthritis (where applicable) and termination.
Figure 10 shows total Ig levels in the mice.
Figure 11 shows the levels of lymph node cellular activation in response to the mitogens Concanavalin A and LPS, and the antigen type II collagen.
Figure 12 shows the response to Con A, LPS and CII in spleen cells.
Figure 13 is a mCT image of joints and bone in normal mice.
Figure 14 is a mCT image of joints and bone in CIA mice.
Figure 15 is a mCT image of joints and bone in CIA mice treated with T-araC 100 mg/kg for 6 weeks.
Figure 16 is a mCT image of joints and bone in CIA mice treated with methotrexate 9 mg/kg for 6 weeks.
Figure 17 is a graph illustrating mouse joints and bone destruction based on mCT images.
Figure 18 are microphotographs showing H & E histological analysis. (One paw from each mouse was subjected to histopathological assessment. The tissue samples were fixed, decalcified, paraffin-embedded, H&E stained. Each paw was scored for severity of architectural changes and marginal erosion.)
Figure 19 is a graph illustrating severity of arthritis based on H & E staining.

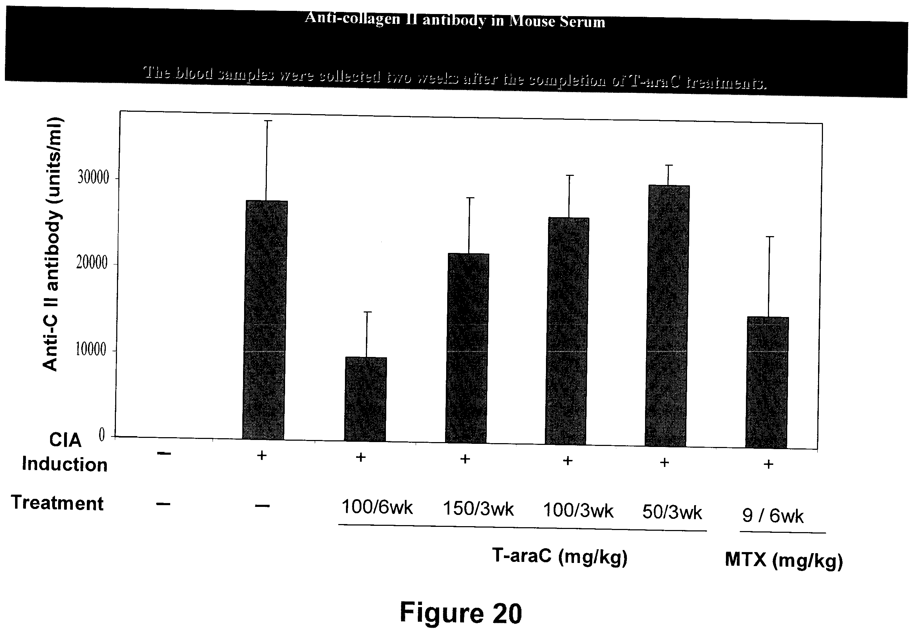

Figure 20 is a graph illustrating anti-collagen antibody in mouse serum. (The blood samples were collected two weeks after the completion of T-araC treatments.)
Figure 21 is a graph illustrating IL-10 in mouse serum.
Figure 22 is a graph illustrating VEGF in mouse serum.

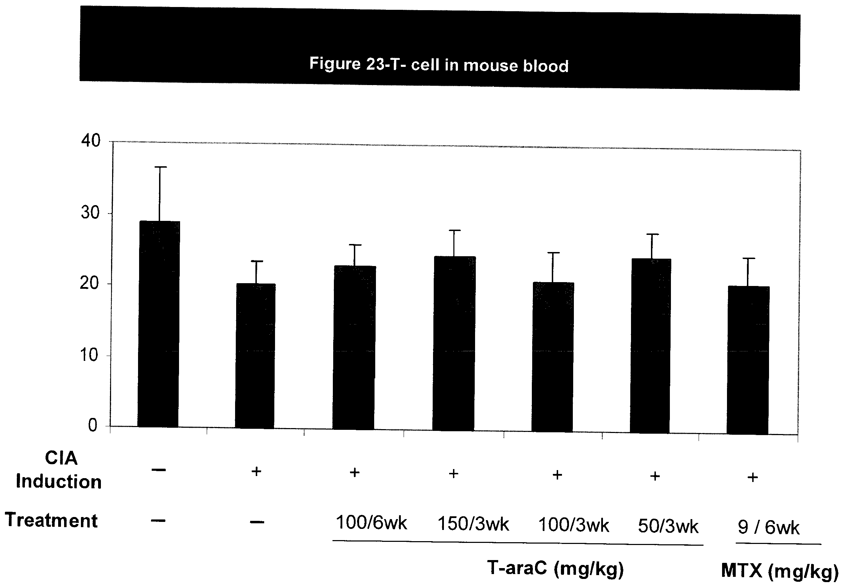

Figure 23 is a graph illustrating T-cell in mouse blood.

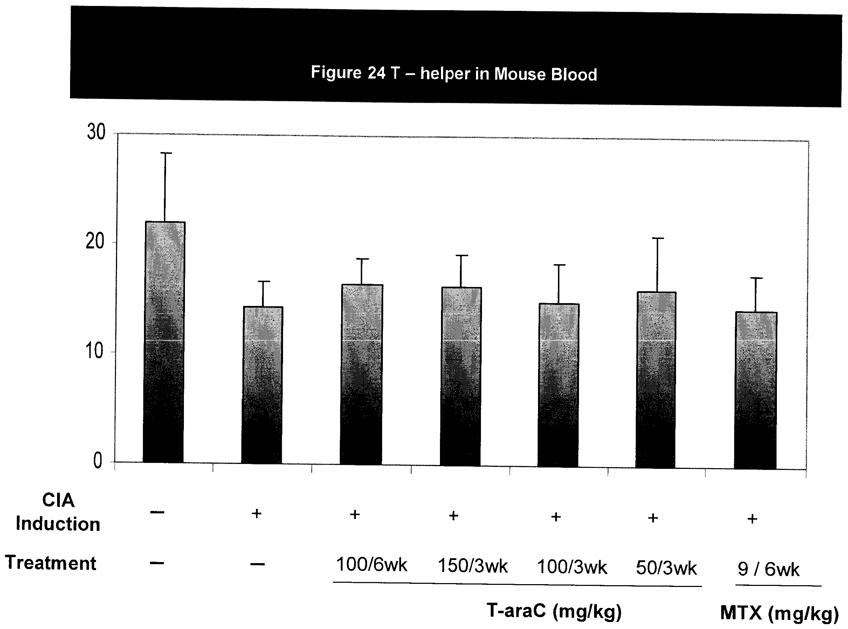

Figure 24 is a graph illustrating T-helper in mouse blood.

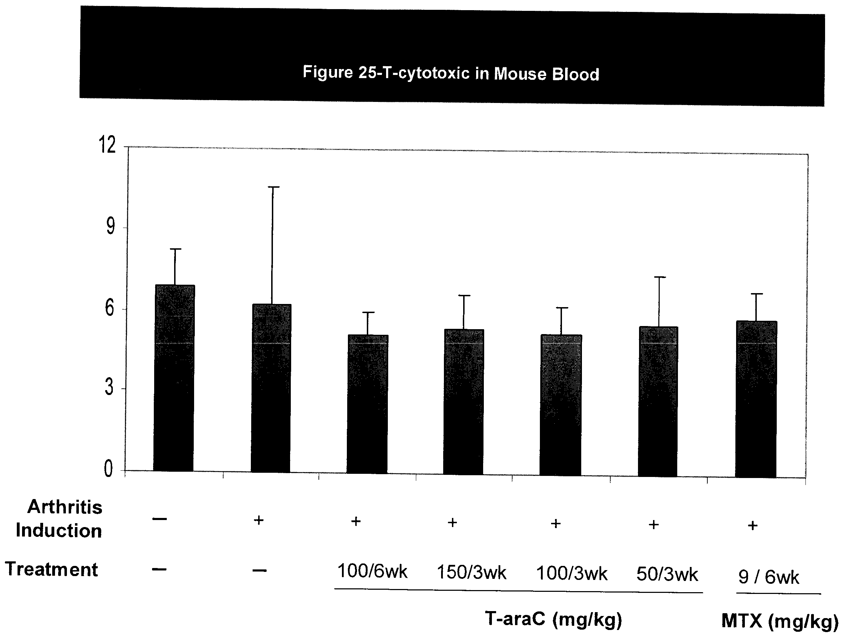

Figure 25 is a graph illustrating T-cytotoxic in mouse blood.

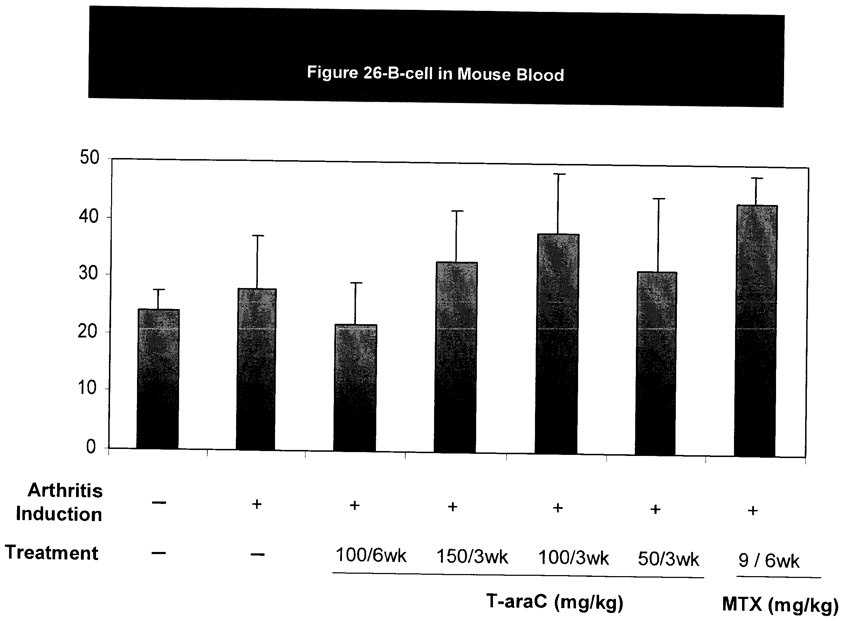

Figure 26 is a graph illustrating B-cell in mouse blood.
Figures 27A-27D illustrate histological findings of collagen-induced arthritis and treatments according to this disclosure.
Figure 28 is a graph showing the influence of treatment according to this disclosure on joint inflammation.
Figure 29 is a graph showing the influence of treatment according to this disclosure on joint erosion.
Figure 30 is a graph showing the influence of treatment according to this disclosure on arthritis pathology.
Figure 31 is a graph showing the influence of treatment according to this disclosure on cartilage matrix loss.

### Best and Various Modes for Carrying Out Disclosure

The present disclosure relates to a method for treating or preventing arthritis which comprises administering to a host in need thereof an amount effective for treating or preventing rheumatoid arthritis of at least one compound represented by the formula 1: 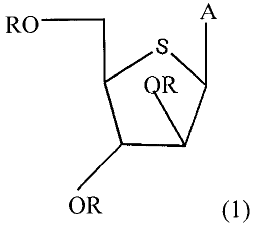
wherein each R individually is H, an aliphatic acyl group or an aromatic acyl group;
A is selected from the group consisting of 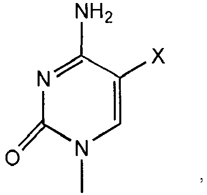 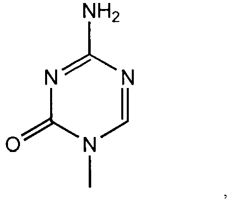 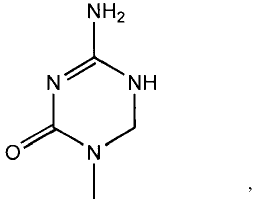 and 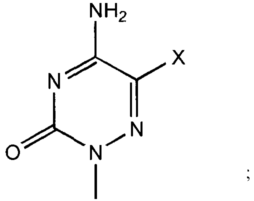 wherein X is selected from the group consisting of hydrogen, halo, alkoxy, alkyl, haloalkyl, alkenyl, haloalkenyl, alkynyl, amino, monoalkylamino, dialkylamino, cyano and nitro.

Each R in formula 1 individually is preferably H or an aliphatic or aromatic acyl group. Typical aliphatic acyl groups contain from 1 to 6 carbon atoms and include formyl, acetyl, and propionyl. Typical aromatic acyl groups include unsubstituted and alkyl substituted aromatic groups containing 7-10 carbon atoms in the aromatic group. When substituted, the alkyl group typically contains 1-6 carbon atoms. Typical aromatic acyl groups include benzoyl and para-toloyl.

Examples of monoalkylamino groups for X contain 1-6 carbon atoms and include monomethylamino, monoethylamino, mono-isopropylamino, mono-n-propylamino, mono-isobutyl-amino, mono-n-butylamino and mono-n-hexylamino. The alkyl moiety can be straight or branched chain.

Suitable dialkylamino groups for Y and X contain 1-6 carbon atoms in each alkyl group. The alkyl groups can be the same or different and can be straight or branched chain. Examples of some suitable groups are dimethylamino, diethylamino, ethylmethylamino, dipropylamino, dibutylamino, dipentylamino, dihexylamino, methylpentylamino, ethylpropylamino and ethylhexylamino.

Examples of halogen groups for X include Cl, Br, F and I with F as the most typical.

Examples of alkyl groups for X typically contain 1-6 carbon atoms and can be straight or branched chain. Some examples are methyl, ethyl, i-propyl, n-propyl, i-butyl, n-butyl, pentyl and hexyl.

Examples of haloalkyl groups typically contain 1-6 carbon atoms and can be straight or branched chain and include Cl, Br, F or Isubstituted alkyl groups including the above specifically disclosed alkyl groups.

Examples of alkoxy groups typically contain 1-6 carbon atoms and include methoxy, ethoxy, propoxy and butoxy.

Examples of alkenyl groups typically contain 2-6 carbon atoms and include ethenyl and propenyl.

Examples of haloalkenyl groups typically contain 1-6 carbon atoms and include Cl, Br, F or I substituted alkenyl groups including the above specifically disclosed alkenyl groups.

Examples of alkynyl groups typically contain 1-6 carbon atoms and include ethynyl and propynyl.

The preferred compounds employed according to the process of the present disclosure are 1-(4-thio-β-D-arabinofuranosyl) fluorocytosine and 1-(4-thio-ß-D-arabinofuranosyl) cytosine, also referred to herein as Thio AraC, and T AraC.

The compounds employed according to the present disclosure can be prepared by the improved process disclosed in US Patent 6,576,621.

Examples of types of arthritis to which the present disclosure is addressed include rheumatoid arthritis and osteoarthritis.

The host treated according to this disclosure includes mammals such as humans and companion animals (e.g. dogs and cats).

The following non-limiting examples illustrate the present disclosure and demonstrate the effectiveness of compounds employed according to this disclosure in treating and preventing arthritis.

The data below reveals a marked anti-arthritic influence of the tested compound (i.e. 1-(4-thio-β-D-arabinofuranosyl) cytosine) in collagen-induced arthritis using a prophylactic protocol. The drug exhibited a strong dose-dependent effect, with complete protection from the onset of disease achieved at 60mg/kg/day, and a significant reduction in disease incidence in mice treated with 20mg/kg/day. While the precise mechanism of action cannot be determined from the current experiment, the drug exerted an effect on the progression of disease, significantly reducing the number of affected paws in mice than did develop arthritis, and also reduced the severity of arthritis in paws with joint disease. A marked reduction of anti-CII antibody titers is also likely to be key in the anti-arthritic activity observed in this study. However, it should be noted that overall immunoglobulin levels also fell, which is indicative of a generalized immunosuppressive effect. A marked reduction in the response to the T cell mitogen Con A was seen in lymph node cells, and a generalized reduction in lymphocyte activity was indicated by the media cell activation responses. Some level of drug toxicity was observed both within the 20 mg/kg/day dose (with a non-lethal skin rash in 20% of mice) and the 60 mg/kg/day/dose (20% skin rash with one death, and marked splenomegaly). Overall, the tested compound exhibited highly promising pre-clinical findings strongly indicating anti-arthritic activity.

Collagen arthritis is induced by immunization of susceptible strains of mice with type II collagen, the major component of joint cartilage (1). A progressive, inflammatory arthritis develops in the majority of immunized animals, which is characterized clinically by erythema and edema, with affected paw width increases of typically 100%. A clinical scoring index has been developed to assess disease progression to joint distortion and spondylitis (2). Histopathology of affected joints reveals synovitis, pannus formation, and cartilage and bone erosion, which may also be represented by an index. Immunological laboratory findings include high antibody levels to type II collagen, and hypergammaglobulinemia. This model is now well established for testing of immunotherapeutic approaches to joint disease (3), and has been successfully employed for the study of both biological and pharmacological agents for the treatment of rheumatoid arthritis (RA) (4;5). This experiment evaluated the influence of the tested compound on CIA using a prophylactic protocol using three doses administered from the time of immunization with type II collagen.

The following materials and methods were used in the testing.

Animals and Compound Administration: Forty DBA/1 LacJ mice 8-10 weeks of age were obtained from Jackson Labs, and acclimatized in the test facility for 10 days prior to experimentation. All animals weighed > 16 grams at the start of the testing. The dosing solution was made fresh weekly and stored at 4°C. Mice were divided into one of four treatment groups:
Group 1. 100 µl sterile vehicle (saline containing 0.05% Tween 80) by daily i.p. injection.
Group 2. 100 µl sterile vehicle containing Compound at 5 mg/kg/day by i.p. injection.
Group 3. 100 µl sterile vehicle containing Compound at 20 mg/kg/day by i.p. injection.
Group 4. 100 µl sterile vehicle containing Compound at 60 mg/kg/day by i.p. injection.

Three days after the initial dosing, all mice were injected with 100 µg bovine type II collagen in Freund's complete adjuvant (FCA) intradermally at the base of the tail. Mice were monitored by daily examination for the onset of disease, which was recorded. Mice were weighed weekly, and overall health status noted. Arthritis affected animals were clinically assessed five times per week until ten weeks after immunization, and paw measurements were made three times per week. Mice without signs of arthritis ten weeks after immunization were considered disease negative.

Immunological assessment: All mice were pre-bled prior to the start of the trial, subsequently at two weeks post immunization, onset of arthritis (where applicable) and at the completion of the trial. Sera were separated and stored at -80°C. ELISA assays were performed to determine (1) anti-type II collagen antibody levels and (2) total immunoglobulin levels. Spleen and lymph nodes were removed at sacrifice, and single cell suspensions prepared. Mitogen responses to Con A and LPS, and antigen proliferative responses to type II collagen were determined using standard techniques.

Analysis: Appropriate statistical comparisons were performed to assess the influence of the compound on (i) disease incidence, (ii) time of disease onset, (iii) individual paw swelling, and (iv) disease progression based on cumulative arthritis score. The immunological data was analysed to examine the influence of the compound on (i) the antibody response to type II collagen (ii) overall immunoglobulin levels (iii) T cell mitogen responses, (iv) B cell mitogen responses, and (v) antigen specific (collagen) proliferative responses

### RESULTS

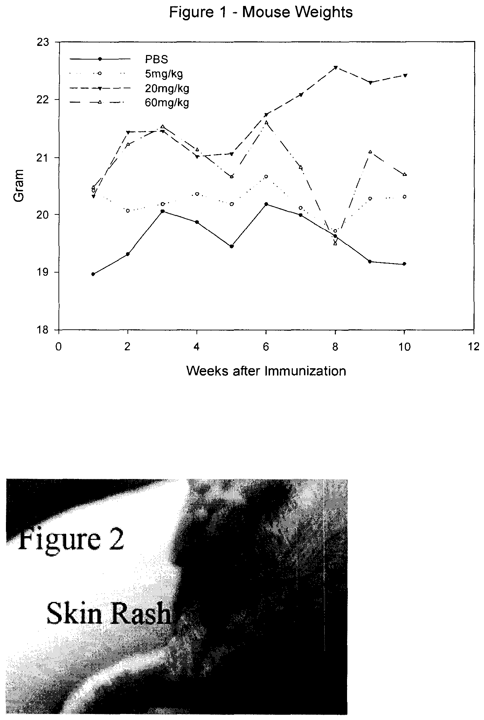

Adverse Effects and Toxicity. Mean mouse weights over the trial period are shown in Figure 1. Despite random group assignment, mice in the control group were non-significantly less heavy than those in the treatment group at the start of the experiment, and their weight subsequently declined following the onset of disease, which is typical in collagen arthritis. A similar weight pattern was observed in mice treated with the test compound at 5 mg/kg/day. In contrast, mice treated with the test compound at 20 mg/kg/day gained weight throughout the trial, at a rate that is similar to normal animals. This resulted in a significant difference between control mice and this treatment group from Week 5 onwards. Mice treated with 60 mg/kg/day gained weight at a normal rate through Week 6, but then lost weight rapidly between Week 6 and Week 8, after which their weight was restored to near normal levels.

One animal in the high dose (60 mg/kg/day) group was euthanized during the study due to the development of a marked ulcerating skin rash exhibiting signs of secondary infection. A milder skin rash was observed in another mouse within the 60 mg/kg/day group, and in two animals in the 20 mg/kg/day group (Figure 2). These manifestations were controlled by cessation of therapy for 3-4 days, after which the skin appeared less irritated, and therapy was restarted without the condition worsening. By the completion of the trial, the rash had typically resolved, leaving an area of mild fibrosis. Skin biopsies were obtained from the affected sites, and the pathology findings are pending.

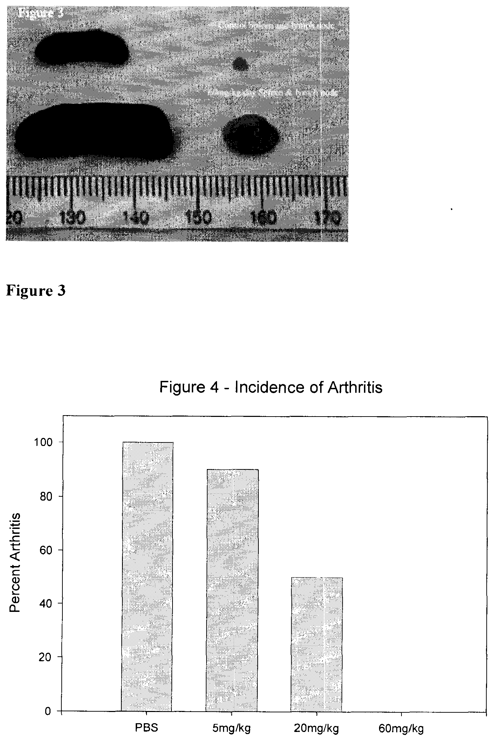

Inspection during necropsy revealed marked splenomegaly in all mice in the high dose (60 mg/kg/day) group (Figure 3), and the spleen weights prior to tissue homogenization indicated an increase of approximately five fold over the PBS control group.. Lymphadenopathy was also observed in some mice, but at a lower incidence and magnitude than the observation on the spleen. This effect was not observed in the lower dose treatment groups. Overall, at 20 mg/kg/day and 5 mg/kg/day the drug was fairly well tolerated, and all mice in these groups survived the study period.

Incidence and Onset of Arthritis- The incidence of arthritis in mice treated with the test compound according to this disclosure at various doses is shown in Figure 4. The most salient feature of the study was the complete protection of mice treated at 60 mg/kg/day, with 0/10 animals with disease compared with 100% incidence (10/10) in the PBS control group (p<0.001). A highly significant reduction in disease incidence (p<0.01) was also observed in mice treated with the test compound at 20 mg/kg/day, while disease incidence (9/10) in mice receiving 5 mg/kg/day was not significantly different from control. The data indicate a clear dose dependant effect upon the incidence of collagen-induced arthritis.

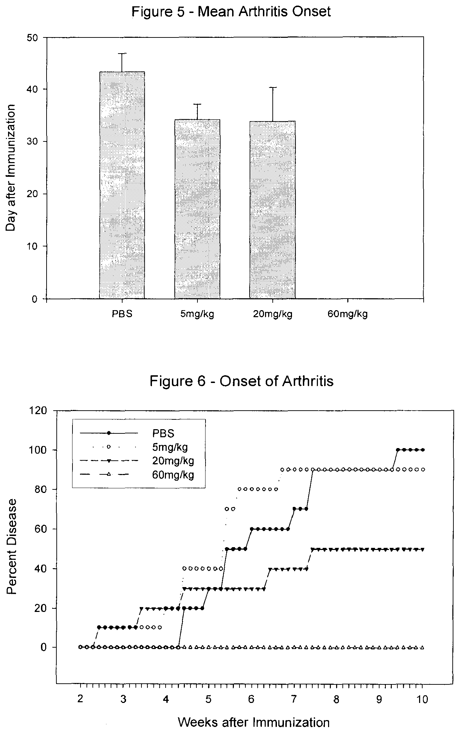

The mean onset of collagen-induced arthritis in the various treatment groups is shown in Figure 5. No significant difference in disease onset was observed between the groups of mice that exhibited arthritis. Although mice in the control group developed disease with a mean onset slightly slower than the treated mice (43 days vs. 34 days), these differences were not statistically significant.

The incidence of arthritis over time is shown in Figure 6. The rate of onset between the treated mice and the control animals is relatively equivalent during the first five week of the trial. From this point on, the rate of onset is considerably slowed in the 20 mg/kg group (but not the 5 mg/kg/day group). The significant reduction in disease incidence seen in the 20 mg/kg group is apparent from Day 50 through the completion of the trial, and the final incidence of disease at the termination of the experiment was significantly different (p<0.01) from the control group

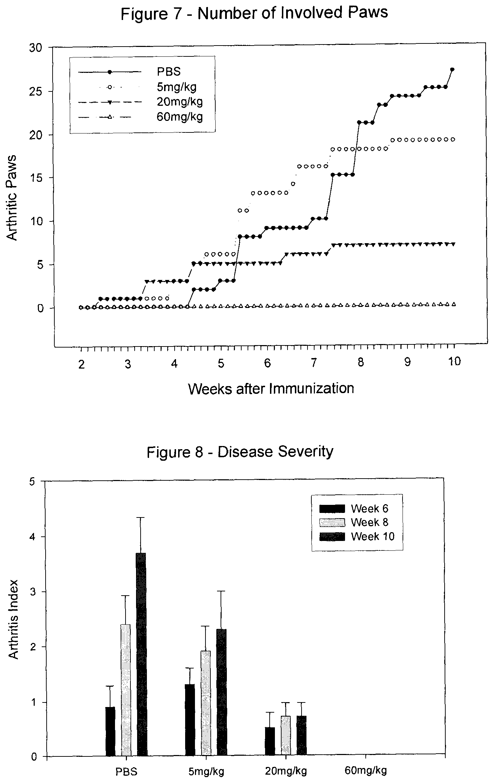

Disease Severity and Progression-Analysis of the cumulative number of arthritic paws (Figure 7) in treated and control animals revealed significant effects of therapy with the test compound on the progression of collagen-induced arthritis. In PBS treated control mice, a total of 27 of 40 paws exhibited signs of arthritis, which was highly significantly different (p<0.001) from mice treated with either 60 mg/kg/day (0/40) or 20 mg/kg/day (7/40). The number of involved paws in mice treated at 5 mg/kg/day was reduced (19/40) compared with control, but this did not reach statistical significance.

Similar findings were observed during the analysis of mean disease severity, shown in Figure 8. The marked worsening of the disease seen from week 6 to the completion of the trial in control mice is typical of collagen arthritis. The advancement of disease severity was clearly checked in mice treated with 20 mg/kg/day, with a significant reduction (p<0.04) observed at week 6, and this difference becoming highly significant (p<0.001) from week 8 to completion of the trial. The reduction of disease severity was less obvious in mice treated with 5 mg/kg/day, but did reach a significant reduction (p<0.05) observed at the completion of the trial (week 10).

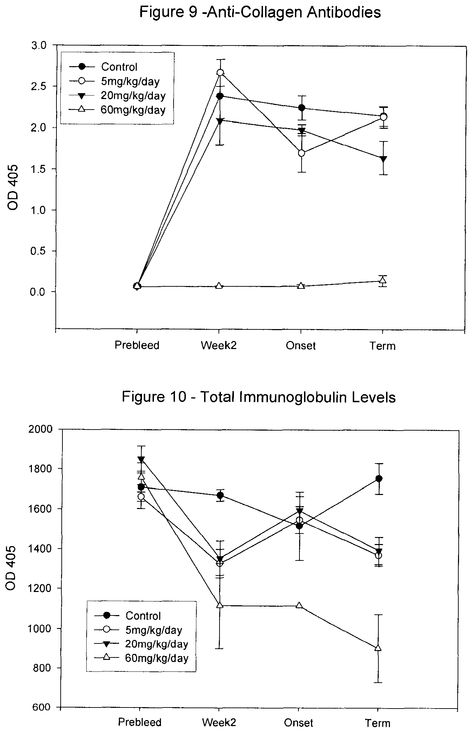

Anti-Type II Collagen Antibody Levels-The levels of anti-CII antibody in sera taken prior to the experiment, 14 days after immunization, onset of arthritis (where applicable) and termination are shown in Figure 9.

Anti-CII titers were completely suppressed in mice treated with the T-araC at 60 mg/kg/day, which provides an indication of the MoA of the anti-arthritic activity. Anti-collagen antibodies were also significantly reduced (p<0.02) in mice receiving 20 mg/kg/day below the levels observed in control mice at the termination of the experiment, and a significant reduction (p<0.01) was observed in mice receiving 5 mg/kg/day at the time of disease onset.

Total Immunoglobulin Levels-Total Ig levels in the mice are shown in Figure 10. There were no significant differences between the groups before the start of the experiment (prebleed). Ig levels in all treated groups fell below control levels at two weeks post treatment, achieving statistical reductions at 5 mg/kg/day, (p<0.05), 20 mg/kg/day, (p<0.05), and 60 mg/kg/day, (p<0.005). However, there was no significant difference between mice in any group at the onset of disease. At the termination of the study, Ig levels in mice treated at 60 mg/kg/day were very low, and levels in mice treated at either 5 mg/kg/day or 20 mg/kg/day were significantly (p<0.02) reduced below control mice.

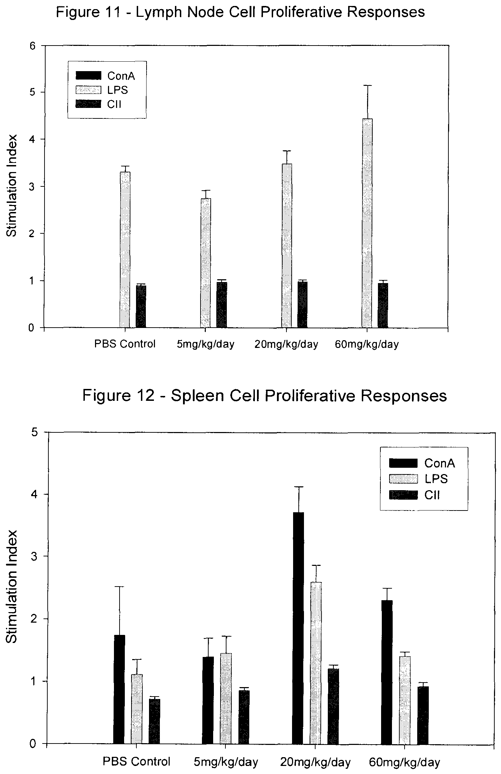

Mitogen and Antigen Proliferative Responses. The levels of lymph node cellular activation in response to the mitogens Concanavalin A and LPS, and the antigen type II collagen are shown in Figure 11. The response to the T cell mitogen concanavalin A was significantly reduced in groups treated with T-araC at 5 mg/kg/day (p<0.04) and 60 mg/kg/day (p<0.001), and approached significance (p=0.08) in mice receiving 20 mg/kg/day. Interestingly, the response to LPS (a predominantly B cell mitogen) was not significantly influenced. The response to the stimulating antigen (type II collagen) was also not affected by treatment.

The response to Con A, LPS and CII in spleen cells is shown in Figure 12. In contrast to the suppression observed in lymph node cells, cellular stimulation appeared to be significantly elevated (p<0.005) in mice treated with the test compound at 20 mg/kg/day. However, examination of the data reveals that the media (non-stimulated) cellular activation levels in mice treated at 20 mg/kg/day (0.53) and 60 mg/kg/day (0.58) are lower than cellular activation levels in control mice (0.77), and this difference is statistically significant (p<0.001 and p<0.05 respectively). Therefore, this apparent increase in the stimulation index is mathematical, due to a reduction in base-line cellular activity, rather than an increase in proliferation.

The above results reveal a marked anti-arthritic influence of the test compound according to the present disclosure in collagen-induced arthritis using a prophylactic protocol. The drug exhibited a strong dose-dependent effect, with complete protection from the onset of disease achieved at 60 mg/kg/day, and a significant reduction in disease incidence in mice treated with 20 mg/kg/day. The drug exerted a marked effect on the progression of disease, significantly reducing the number of affected paws in mice than did develop arthritis, and also reduced the severity of arthritis in paws with joint disease. While the precise mechanism of action cannot be determined from the current experiment, the marked reduction of anti-CII antibody titers is likely to be key in the anti-arthritic activity observed in this study. However, it should be noted that overall immunoglobulin levels also fell, which is indicative of a generalized immunosuppressive effect, rather than a specific reduction in the autoimmune activity. A marked reduction in the response to the T cell mitogen Con A was seen in lymph node cells, and a generalized reduction in lymphocyte activity was indicated by the media cell activation responses. Some level of drug toxicity was observed both within the 20 mg/kg/day dose (with a non-lethal skin rash in 20% of mice) and the 60 mg/kg/day/dose (20% skin rash with one death, and marked splenomegaly). Overall, the drug exhibited highly promising pre-clinical findings strongly supporting anti-arthritic activity.

In further testing, mice were injected with collagen II/complete Freunds adjuvant (CFA) on Day 0 as the primary immunization and injected again with collagen II without CFA on Day 21 as a booster injection. Treatment of 6 weeks started on Day -2 and treatment of 3 weeks started on Day 19. All treatments were finished on Day 40. Tissue and blood samples were collected on Day 54 for pathological and immunological analyses.

**Study Design for Evaluation of T-araC Activity with CIA Mouse Model**

| Mouse Group | Number of Animal | Treatment (IP) | Dose (mg/kg/inj) | Treatment Schedule | Treatment duration |
|---|---|---|---|---|---|
| 1 | 10 | Vehicle | 0 | M/W/F | 6 wk |
| 2 | 10 | T-araC | 100 | M/W/F | 6 wk |
| 3 | 10 | T-araC | 50 | M/W/F | 6 wk |
| 4 | 10 | T-araC | 25 | M/W/F | 6 wk |
| 5 | 10 | T-araC | 150 | M/W/F | 3 wk |
| 6 | 10 | T-araC | 100 | M/W/F | 3 wk |
| 7 | 10 | T-araC | 50 | M/W/F | 3 wk |
| 8 | 10 | MTX | 9 | Q2Dx22 | 6 wk |

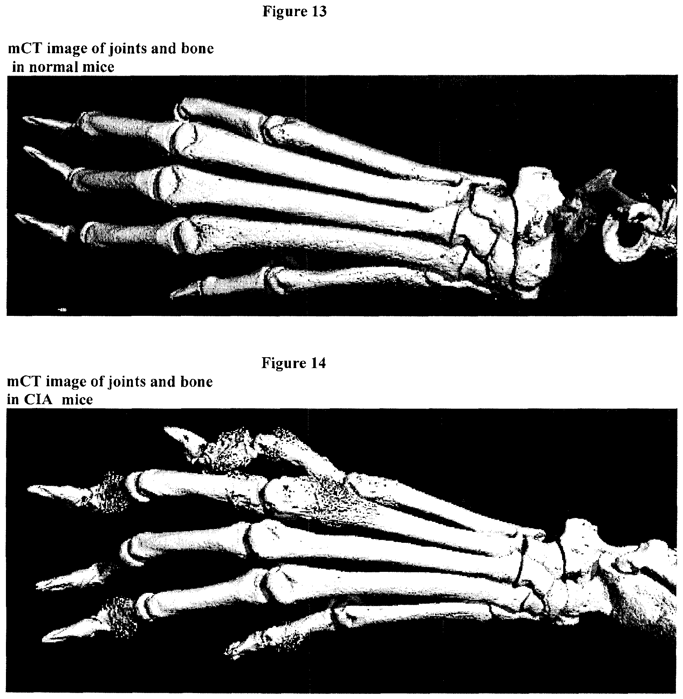

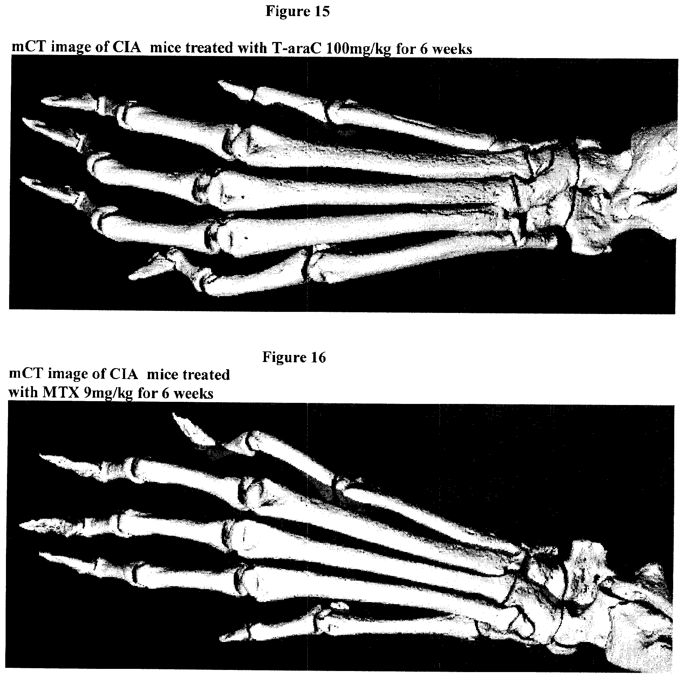

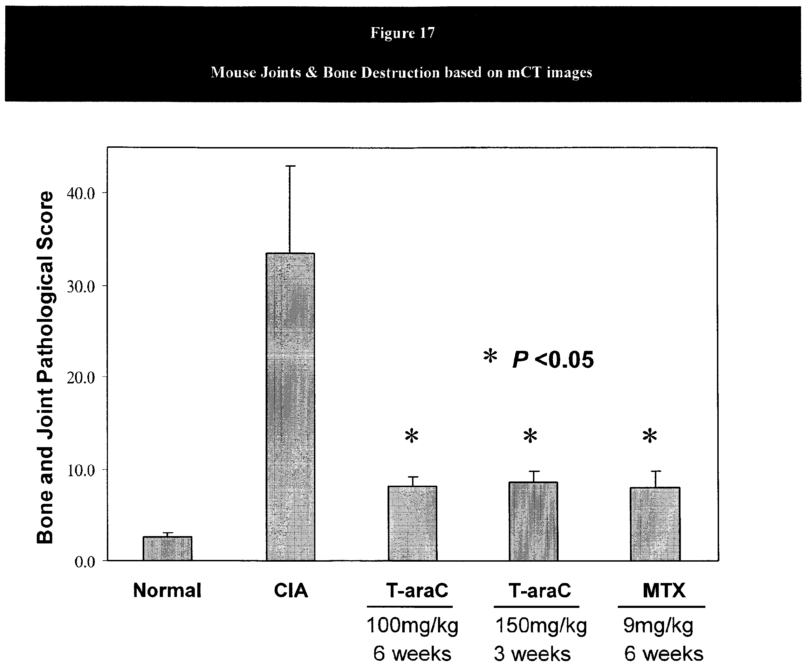

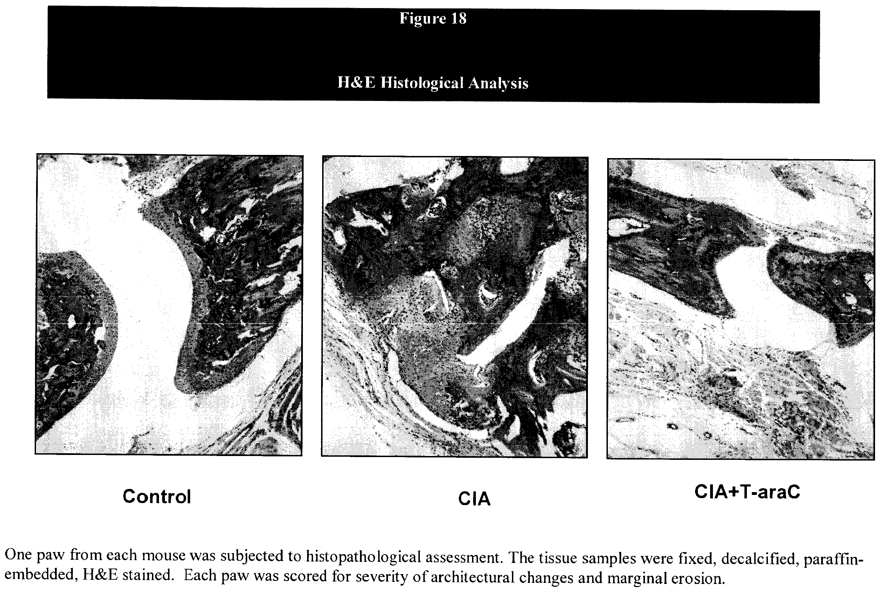

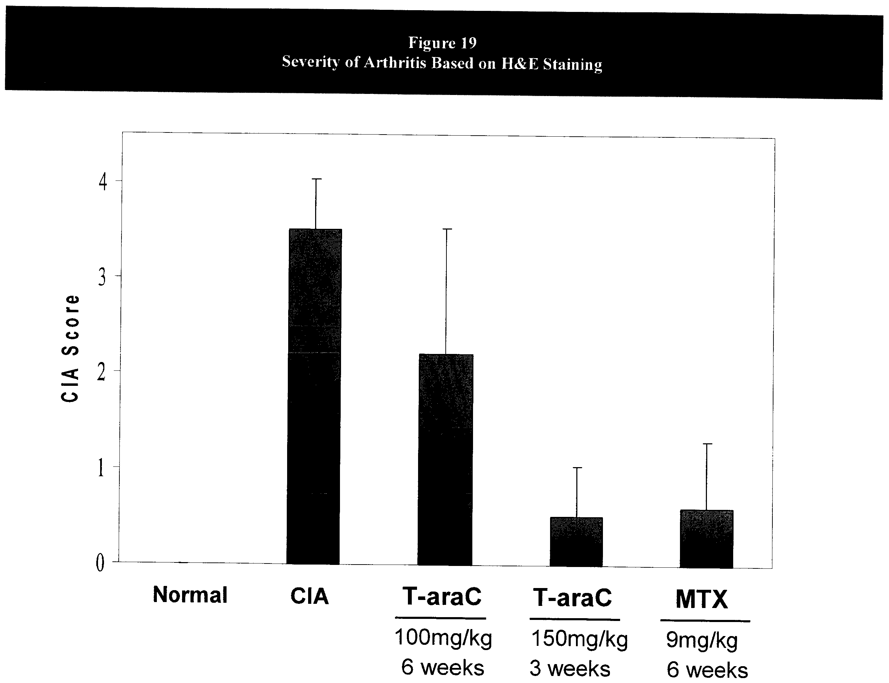

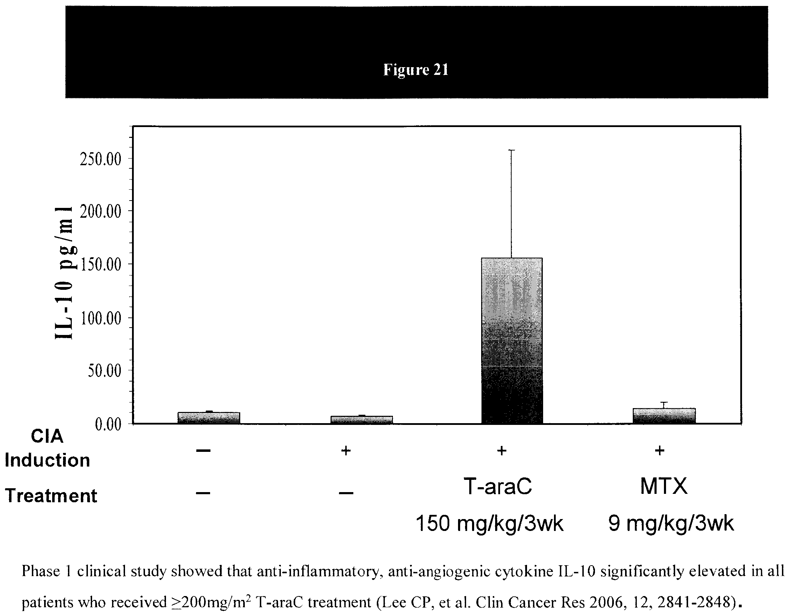

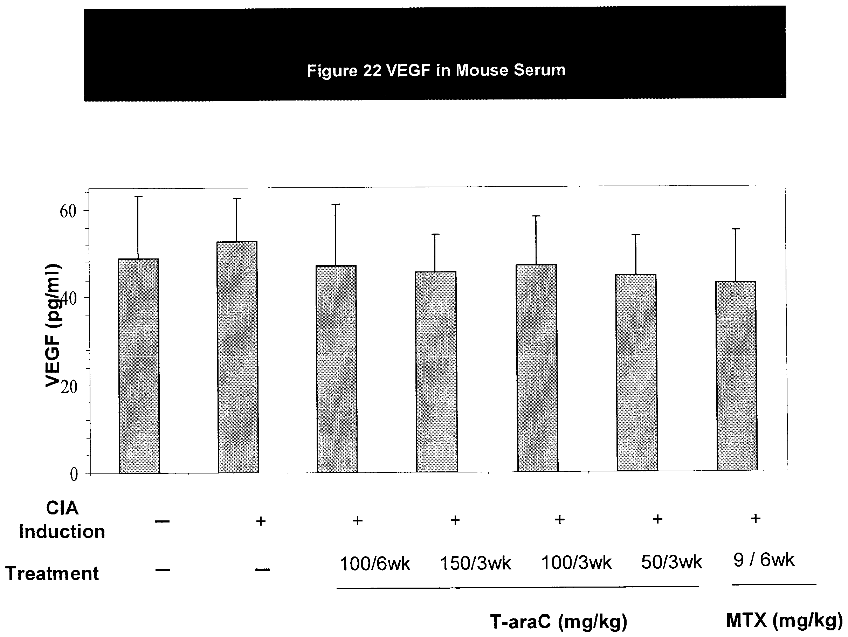

X-ray microtomograph (mCT) provides non-destructive 3-dimensional internal microscopy for detection of bone and joint destruction and erosions in mouse. The technology was used to study T-araC [1-(4-thio-β-D-arabinofuranosyl) cytosine] drug efficacy in treatment of arthritis. For example see figures 13-16 which are mCT images of joints and bone in normal mice, CIA mice, CIA mice treated with T-araC and CIA mice treated with methotrexate, respectively. The micro CT images of 3 dimensional internal microscopy and the quantitative analysis of joint and bone erosion (Figure 17) demonstrated the significant anti-arthritis activity of T-araC with two treatment dosages and schedules. T-araC showed similar activity in the CIA model as methotrexate, a current clinically used drug against rheumatoid arthritis. Histological analysis of mouse paw has been conducted for assessing inflammatory and erosive changes around mouse joins. The results also showed significant anti-arthritis activity of T-araC (Figure 18 and 19), consisting with the micro CT analysis. In addition, consisting with the Phase I clinical study, anti-inflammatory and anti-angiogenic cytokine IL-10 significantly elevated in mouse blood serum after T-araC treatment (Figure 21). Vascular Endothelial Growth Factor (VEGF) content in mouse serum was examined with ELISA and no significant difference were observed between different treatment groups (Figure 22). T-cell and B-cell in mouse blood samples were counted at the time point of two weeks after T-araC treatments and no reduction of neither cell types was found, indicating if there was any immunosuppressive effect on T-cell or B-cell counts associated with T-araC treatments that might be reversible. In collusion, this pre-clinical study with the CIA mouse model and the treatment design showed in the table demonstrated that the anti-arthritis activity of T-araC is statistically significant while no obvious toxicity associated with T-araC treatments was observed.

In still further testing sixty DBA/1 LacJ mice 8-10 weeks of age were obtained and acclimatized in the test facility for a minimum of 10 days prior to experimentation. All animals weighed >16grams at the start of the experiment. Mice were injected with 100 µg bovine type II collagen in Freund's complete adjuvant (FCA) intradermally at the base of the tail, and monitored by daily examination for the onset of disease, which was recorded. At the first appearance of clinical evidence of arthritis, mice were divided into one of four treatment groups:

| | |
|---|---|
| Group 1. | 100 µl sterile vehicle by oral gavage x3 per week. |
| Group 2. | 100 µl sterile vehicle containing T-araC at 30 mg/kg by oral gavage. |
| Group 3. | 100 µl sterile vehicle containing T-araC at 60 mg/kg by oral gavage. |
| Group 4. | 100 µl sterile vehicle containing T-araC at 90 mg/kg by oral gavage. |

Mice were weighed weekly, and overall health status noted. Animals were clinically assessed for disease five times per week until ten weeks after disease onset, and paw measurements were made three times per week.

Histological Assessment. Limbs from all mice were removed at the completion of the clinical assessment study, and stored in neutral buffered formalin solution. Joints were decalcified for 18 days in 10% formic acid, dehydrated, and embedded in paraffin blocks. Sections were cut along a longitudinal axis, mounted and stained with either hematoxylin and eosin or Toluidine Blue. Specimens were cut to approximately the mid line, and then sagital central samples mounted for evaluation. This allowed for a consistent geographic evaluation. Five to ten samples were mounted (usually 4 - 6 samples per slide). After staining, the slides were permanently bonded with coverslips. A minimum of 3 separate sections per specimen were evaluated in a blinded fashion, with the evaluated unaware of the group assignment. On front limbs, all elbow, wrist, and metacarpal joints were scored, while all knee, ankle, and metatarsal joints were scored on the rear paws. Digits were not evaluated, since the sectioning procedure eliminates most PIP joints. Slides were evaluated for the presence of synovitis, pannus formation, marginal erosions, architectural changes (mostly subluxation), and destruction. An overall score, based on these collective points, was then assigned to each section. The scoring system was based as follows:

**Synovitis was judged by the thickness of the synovial membrane, and scored:**

| | | |
|---|---|---|
| 0 | - | less than 3 cells thick |
| 1 | - | 3 - 5 cells thick |
| 2 | - | 6 - 10 cells thick |
| 3 | - | 10 - 20 cells thick |
| 4 | - | 20 - 30 cells thick |

**Pannus formation was scored as follows:**

| | | |
|---|---|---|
| 0 | - | No pannus formation |
| 1 | - | Microvillus present |
| 2 | - | Clear pannus attachment |
| 3 | - | Marked pannus attachment |
| 4 | - | Joint space filled by pannus |

**Marginal erosions were scored as follows:**

| | | |
|---|---|---|
| 0 | - | No erosions visible |
| 1 | - | minor indentation in area of capsular attachment |
| 2 | - | Clear erosions of cartilage |
| 3 | - | Erosions extend into subchondral bone |
| 4 | - | Major erosion of bone and cartilage |

**Architectural changes were scored as follows:**

| | | |
|---|---|---|
| 0 | - | Normal joint architecture |
| 1 | - | Edematous changes |
| 2 | - | Minor subluxation of articulating surfaces |
| 3 | - | Major subluxation of articulating surfaces |
| 4 | - | Complete fibrosis and collagen bridging |

**The overall score reflected:**

| | | |
|---|---|---|
| 0 | - | Classical normal joint appearance |
| 1 | - | Minor changes; consistent with remission; may be clinically normal. |
| 2 | - | Definite inflammatory arthritis |
| 3 | - | Major inflammatory, erosive disease |
| 4 | - | Destructive, erosive arthritis |

The toluidine blue sections were evaluated for proteoglycan loss. The staining at the articular surface was compared to staining at the growth plate, and was scored as follows:
0 - No proteoglycan loss; Normal Toluidine Blue staining.
1 - Minor proteoglycan loss; Some loss of staining from the superficial cartilage
2 - Moderate proteoglycan loss; Weak staining of superficial cartilage
3 - Significant proteoglycan loss; No Toluidine Blue staining of superficial cartilage
4 - Major proteoglycan loss; No Toluidine Blue staining of deep cartilage

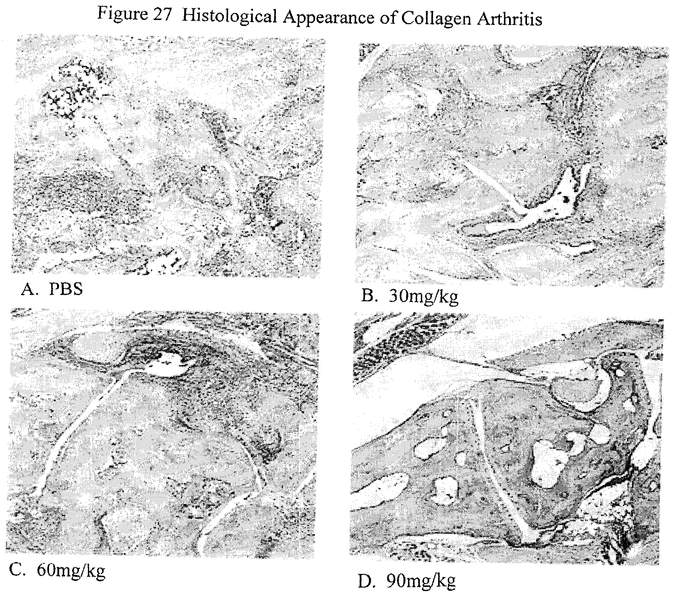

Histological Findings of Collagen-induced arthritis. Sections were assessed for the inflammatory and erosive parameters of disease. The appearance of the arthritis (Figure 27A) reveals severe inflammatory erosive disease pathology in the control (PBS treated) group, with the typical arthritic features of synovial hypertrophy and hyperplasia, with marked pannus attachment and marginal erosions.

Treatment with the T-araC compound at 30 mg/kg/day (Figure 27B) resulted in significant changes in the arthritis parameters, with reductions in both inflammatory and erosive joint changes. Treatment with T-araC at 60 mg/kg/day (Figure 27C) resulted in a more marked reduction in pannus formation and erosions compared with the control, and administration of T-araC at 90 mg/kg/day (Figure 27D) resulted in the appearance of minor changes or a normal joint with a thin synovial membrane, smooth cartilage surfaces and normal bone. In addition, in these studies with the various doses of the treatments with the T-araC no skin rashes were reported by the animal technicians.

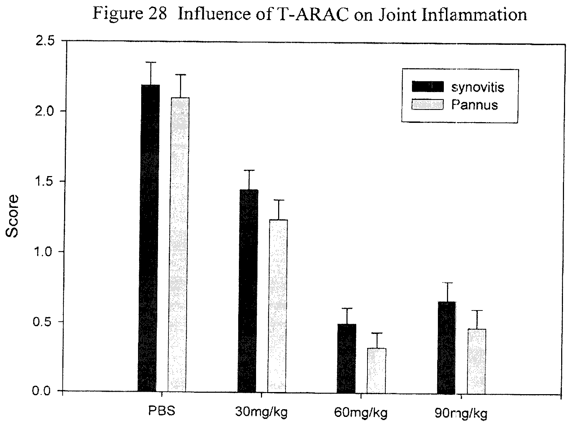

Analysis of the inflammatory scores (Figure 28) revealed a dose-dependant reduction in the inflammation in mice treated with T-araC when compared with control (saline-treated) animals. The synovitis was significantly reduced (p<0.01), and the pannus formation showed similar reductions in score (p<0.01) in mice treated with T-araC at 30 mg/kg. These parameters where highly significantly reduced (p<0.001) in mice treated at with 60 mg/kg or 90 mg/kg, and the higher dose groups were also significantly different for mice receiving T-araC at 30 mg/kg.

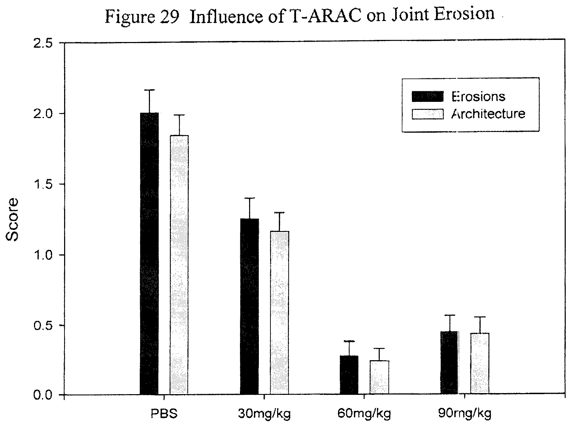

Assessment of changes in the erosive features (erosions and changes in joint architecture) of collagen-induced arthritis showed a similar pattern of effects. Highly significant reductions (p<0.001) in joint erosions were observed between the group treated with T-araC at 30 mg/kg/day and 90 mg/kg/day when compared with control (saline-treated) animals (Figure 29), and significant reductions (p<0.01) were observed in mice treated with T-araC compound at 20 mg/kg/day.

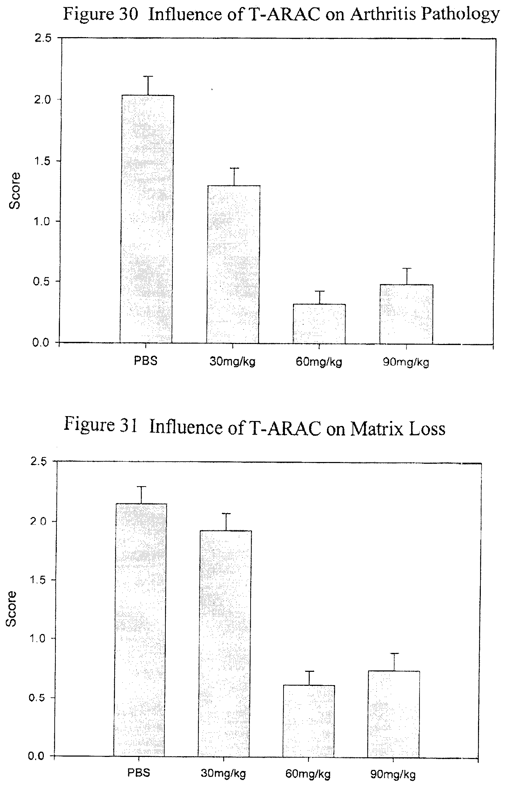

The combination of the histopathological parameters into an overall histological arthritis score (Figure 30) reflected the findings of the individual disease parameters. Significant, dose-dependent differences between mice treated with T-araC and the control (PBS) treated animals observed. The reduction in overall disease in mice treated at 60 mg/kg/day and 90 mg/kg/day was highly significant (p<0.001).

The Toluidine Blue stained sections were examined to determine whether T-araC influenced the loss of matrix proteins from the arthritic joint. The data (Figure 31) indicate that T-araC at 60 mg/kg/day or 90 mg/kg/day did protect against proteoglycan loss, and this effect was highly statistically significant.

The histological findings confirm the clinical data that indicate that treatment of established collagen-induced arthritis with compounds according to the present disclosure using a therapeutic protocol exerted a marked dose-dependant amelioration of disease. The reduction of all histological parameters of arthritis reached high of levels statistical significance in mice treated with either 60 mg/kg or 90 mg/kg. However, a statistical reduction of disease was observed at all doses. At the high doses of the T-araC there was remarkable restoration of the joint structure. The overall impression is that T-araC allowed some degree of repair of joint disease in mice treated at high doses. Overall, these findings are in agreement with clinical observations made, and are very encouraging. The results suggest that compounds of the present disclosure can exert an anti- arthritic effect when administered in a therapeutic manner to established arthritis.

To summarize the therapeutic trial revealed remarkable anti-arthritic effects, with 100% of mice receiving 90 mg/kg/day entering disease remission at some point during the trial, and 60% of animals maintained in a clinically disease free state at the conclusion of the trial. In addition, a highly significant reduction in the arthritis index and number of involved paws were observed. A significant reduction in arthritis was also observed in mice treated at 60 mg/kg/day, with 70% entering disease remission at some point, and 40% remaining in remission at the conclusion of the trial. Again, a significant reduction in the disease score and the number of involved limbs was recorded. No significant effects on clinical disease were observed in mice treated at 30 mg/kg/day. The histological findings confirmed the clinical data and indicated that treatment of established collagen-induced arthritis with T-araC, a compound according to this disclosure, resulted in a marked dose-dependant amelioration of disease. The reduction of all histological parameters of arthritis reached high of levels statistical significance, and at the high doses of T-araC, a remarkable restoration of the joint structure was observed. Investigation of the splenomegaly (which was ubiquitous in all mice treated with T-araC independent of arthritis efficacy) indicated that the most pronounced change was cellular hyperproliferation in the absence of tissue necrosis or fibrosis. This marked cellular increase appeared to account for the expansion in size of the spleen.

### Formulations

The compounds of the present disclosure can be administered by any conventional means available for use in conjunction with pharmaceuticals, either as individual therapeutic agents or in a combination of therapeutic agents. They can be administered alone, but generally administered with a pharmaceutical carrier selected on the basis of the chosen route of administration and standard pharmaceutical practice. The compounds can also be administered in conjunction with other therapeutic agents such as interferon (IFN), interferon α-2a, interferon α-2b, consensus interferon (CIFN), ribavirin, amantadine, remantadine, interleukine-12, ursodeoxycholic acid (UDCA), and glycyrrhizin.

The pharmaceutically acceptable carriers described herein, for example, vehicles, adjuvants, excipients, or diluents, are well-known to those who are skilled in the art. Typically, the pharmaceutically acceptable carrier is chemically inert to the active compounds and has no detrimental side effects or toxicity under the conditions of use. The pharmaceutically acceptable carriers can include polymers and polymer matrices.

The compounds of this disclosure can be administered by any conventional method available for use in conjunction with pharmaceuticals, either as individual therapeutic agents or in a combination of therapeutic agents.

The dosage administered will, of course, vary depending upon known factors, such as the pharmacodynamic characteristics of the particular agent and its mode and route of administration; the age, health and weight of the recipient; the nature and extent of the symptoms; the kind of concurrent treatment; the frequency of treatment; and the effect desired. A daily dosage of active ingredient can be expected to be about 0.001 to 1000 milligrams (mg) per kilogram (kg) of body weight, with the preferred dose being 0.1 to about 30 mg/kg.

Dosage forms (compositions suitable for administration) contain from about 1 mg to about 500 mg of active ingredient per unit. In these pharmaceutical compositions, the active ingredient will ordinarily be present in an amount of about 0.5-95% weight based on the total weight of the composition.

The active ingredient can be administered orally in solid dosage forms, such as capsules, tablets, and powders, or in liquid dosage forms, such as elixirs, syrups and suspensions. It can also be administered parenterally, in sterile liquid dosage forms. The active ingredient can also be administered intranasally (nose drops) or by inhalation of a drug powder mist. Other dosage forms are potentially possible such as administration transdermally, via patch mechanism or ointment.

Formulations suitable for oral administration can consist of (a) liquid solutions, such as an effective amount of the compound dissolved in diluents, such as water, saline, or orange juice; (b) capsules, sachets, tablets, lozenges, and troches, each containing a predetermined amount of the active ingredient, as solids or granules; (c) powders; (d) suspensions in an appropriate liquid; and (e) suitable emulsions. Liquid formulations may include diluents, such as water and alcohols, for example, ethanol, benzyl alcohol, propylene glycol, glycerin, and the polyethylene alcohols, either with or without the addition of a pharmaceutically acceptable surfactant, suspending agent, or emulsifying agent. Capsule forms can be of the ordinary hard- or soft-shelled gelatin type containing, for example, surfactants, lubricants, and inert fillers, such as lactose, sucrose, calcium phosphate, and corn starch. Tablet forms can include one or more of the following: lactose, sucrose, mannitol, corn starch, potato starch, alginic acid, microcrystalline cellulose, acacia, gelatin, guar gum, colloidal silicon dioxide, croscarmellose sodium, talc, magnesium stearate, calcium stearate, zinc stearate, stearic acid, and other excipients, colorants, diluents, buffering agents, disintegrating agents, moistening agents, preservatives, flavoring agents, and pharmacologically compatible carriers. Lozenge forms can comprise the active ingredient in a flavor, usually sucrose and acacia or tragacanth, as well as pastilles comprising the active ingredient in an inert base, such as gelatin and glycerin, or sucrose and acadia, emulsions, and gels containing, in addition to the active ingredient, such carriers as are known in the art.

The compounds of the present disclosure, alone or in combination with other suitable components, can be made into aerosol formulations to be administered via inhalation. These aerosol formulations can be placed into pressurized acceptable propellants, such as dichlorodifluoromethane, propane, and nitrogen. They also may be formulated as pharmaceuticals for non-pressured preparations, such as in a nebulizer or an atomizer.

Formulations suitable for parenteral administration include aqueous and non-aqueous, isotonic sterile injection solutions, which can contain anti-oxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The compound can be administered in a physiologically acceptable diluent in a pharmaceutical carrier, such as a sterile liquid or mixture of liquids, including water, saline, aqueous dextrose and related sugar solutions, an alcohol, such as ethanol, isopropanol, or hexadecyl alcohol, glycols, such as propylene glycol or polyethylene glycol such as poly(ethyleneglycol) 400, glycerol ketals, such as 2,2-dimethyl-1,3-dioxolane-4-methanol, ethers, an oil, a fatty acid, a fatty acid ester or glyceride, or an acetylated fatty acid glyceride with or without the addition of a pharmaceutically acceptable surfactant, such as a soap or a detergent, suspending agent, such as pectin, carbomers, methylcellulose, hydroxypropylmethylcellulose, or carboxymethylcellulose, or emulsifying agents and other pharmaceutical adjuvants.

Oils, which can be used in parenteral formulations include petroleum, animal, vegetable, or synthetic oils. Specific examples of oils include peanut, soybean, sesame, cottonseed, corn, olive, petrolatum, and mineral. Suitable fatty acids for use in parenteral formulations include oleic acid, stearic acid, and isostearic acid. Ethyl oleate and isopropyl myristate are examples of suitable fatty acid esters. Suitable soaps for use in parenteral formulations include fatty alkali metal, ammonium, and triethanolamine salts, and suitable detergents include (a) cationic detergents such as, for example, dimethyldialkylammonium halides, and alkylpyridinium halides, (b) anionic detergents such as, for example, alkyl, aryl, and olefin sulfonates, alkyl, olefin, ether, and monoglyceride sulfates, and sulfosuccinates, (c) nonionic detergents such as, for example, fatty amine oxides, fatty acid alkanolamides, and polyoxyethylene polypropylene copolymers, (d) amphoteric detergents such as, for example, alkyl ß-aminopropionates, and 2-alkylimidazoline quaternary ammonium salts, and (e) mixtures thereof.

The parenteral formulations typically contain from about 0.5% to about 25% by weight of the active ingredient in solution. Suitable preservatives and buffers can be used in such formulations. In order to minimize or eliminate irritation at the site of injection, such compositions may contain one or more nonionic surfactants having a hydrophile-lipophile balance (HLB) of from about 12 to about 17. The quantity of surfactant in such formulations ranges from about 5% to about 15% by weight. Suitable surfactants include polyethylene sorbitan fatty acid esters, such as sorbitan monooleate and the high molecular weight adducts of ethylene oxide with a hydrophobic base, formed by the condensation of propylene oxide with propylene glycol.

Pharmaceutically acceptable excipients are also well-known to those who are skilled in the art. The choice of excipient will be determined in part by the particular compound, as well as by the particular method used to administer the composition. Accordingly, there is a wide variety of suitable formulations of the pharmaceutical composition of the present disclosure. The following methods and excipients are merely exemplary and are in no way limiting. The pharmaceutically acceptable excipients preferably do not interfere with the action of the active ingredients and do not cause adverse side-effects. Suitable carriers and excipients include solvents such as water, alcohol, and propylene glycol, solid absorbants and diluents, surface active agents, suspending agent, tableting binders, lubricants, flavors, and coloring agents.

The formulations can be presented in unit-dose or multi-dose sealed containers, such as ampules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets. The requirements for effective pharmaceutical carriers for injectable compositions are well known to those of ordinary skill in the art. See Pharmaceutics and Pharmacy Practice, J.B. Lippincott Co., Philadelphia, PA, Banker and Chalmers, Eds., 238-250 (1982) *and* ASHP Handbook on Injectable Drugs, Toissel, 4th ed., 622-630 (1986).

Formulations suitable for topical administration include lozenges comprising the active ingredient in a flavor, usually sucrose and acacia or tragacanth; pastilles comprising the active ingredient in an inert base, such as gelatin and glycerin, or sucrose and acacia; and mouthwashes comprising the active ingredient in a suitable liquid carrier; as well as creams, emulsions, and gels containing, in addition to the active ingredient, such carriers as are known in the art.

Additionally, formulations suitable for rectal administration may be presented as suppositories by mixing with a variety of bases such as emulsifying bases or watersoluble bases. Formulations suitable for vaginal administration may be presented as pessaries, tampons, creams, gels, pastes, foams, or spray formulas containing, in addition to the active ingredient, such carriers as are known in the art to be appropriate.

Suitable pharmaceutical carriers are described in Remington's Pharmaceutical Sciences, Mack Publishing Company, a standard reference text in this field.

The dose administered to an animal, particularly a human, in the context of the present disclosure should be sufficient to affect a therapeutic response in the animal over a reasonable time frame. One skilled in the art will recognize that dosage will depend upon a variety of factors including a condition of the animal, the body weight of the animal, as well as the severity and stage of the condition being treated.

A suitable dose is that which will result in a concentration of the active agent in a patient which is known to affect the desired response. The preferred dosage is the amount which results in maximum inhibition of the condition being treated, without unmanageable side effects.

The size of the dose also will be determined by the route, timing and frequency of administration as well as the existence, nature, and extend of any adverse side effects that might accompany the administration of the compound and the desired physiological effect.

Useful pharmaceutical dosage forms for administration of the compounds according to the present disclosure can be illustrated as follows:

### Hard Shell Capsules

A large number of unit capsules are prepared by filling standard two-piece hard gelatine capsules each with 100 mg of powdered active ingredient, 150 mg of lactose, 50 mg of cellulose and 6 mg of magnesium stearate.

### Soft Gelatin Capsules

A mixture of active ingredient in a digestible oil such as soybean oil, cottonseed oil or olive oil is prepared and injected by means of a positive displacement pump into molten gelatin to form soft gelatin capsules containing 100 mg of the active ingredient. The capsules are washed and dried. The active ingredient can be dissolved in a mixture of polyethylene glycol, glycerin and sorbitol to prepare a water miscible medicine mix.

### Tablets

A large number of tablets are prepared by conventional procedures so that the dosage unit was 100 mg of active ingredient, 0.2 mg. of colloidal silicon dioxide, 5 mg of magnesium stearate, 275 mg of microcrystalline cellulose, 11 mg of starch, and 98.8 mg of lactose. Appropriate aqueous and non-aqueous coatings may be applied to increase palatability, improve elegance and stability or delay absorption.

### Immediate Release Tablets/Capsules

These are solid oral dosage forms made by conventional and novel processes. These units are taken orally without water for immediate dissolution and delivery of the medication. The active ingredient is mixed in a liquid containing ingredient such as sugar, gelatin, pectin and sweeteners. These liquids are solidified into solid tablets or caplets by freeze drying and solid state extraction techniques. The drug compounds may be compressed with viscoelastic and thermoelastic sugars and polymers or effervescent components to produce porous matrices intended for immediate release, without the need of water.

Moreover, the compounds of the present disclosure can be administered in the form of nose drops, or metered dose and a nasal or buccal inhaler. The drug is delivered from a nasal solution as a fine mist or from a powder as an aerosol.

The term "comprising" (and its grammatical variations) as used herein is used in the inclusive sense of "having" or "including" and not in the exclusive sense of "consisting only of." The term "consisting essentially of" as used herein is intended to refer to including that which is explicitly recited along with what does not materially affect the basic and novel characteristics of that recited or specified. The terms "a" and "the" as used herein are understood to encompass the plural as well as the singular.

### References

(1) Wooley PH, Luthra HS, Stuart JM, David CS. Type II collagen-induced arthritis in mice. I. Major histocompatibility complex (I region) linkage and antibody correlates. Journal of Experimental Medicine 1981; 154:688-700.
(2) Wooley PH. Collagen-induced arthritis in the mouse. Methods In Enzymology 1988; 162:361-373.
(3) Staines NA, Wooley PH. Collagen arthritis--what can it teach us? British Journal of Rheumatology 1994; 33(9):798-807.
(4) Wooley PH, Whalen JD, Chapman DL, Berger AE, Richard KA, Aspar DG. The effect of an interleukin-1 receptor antagonist protein on type II collagen-induced arthritis and antigen-induced arthritis in mice. Arthritis Rheum 1993; 36:1305-1314.
(5) Wooley PH, Dutcher J, Widmer MB, Gillis S. Influence of a recombinant human soluble tumor necrosis factor receptor FC fusion protein on type II collagen-induced arthritis in mice. Journal of Immunology 1993; 151:6602-6607.

## Claims

1. Compound 1-(4-thio-[beta]-D-arabinofuranosyl) cytosine for use in a method for treating or preventing rheumatoid arthritis which comprises administering to a host in need thereof an amount of compound effective for treating or preventing rheumatoid arthritis.

2. The compound for use according to claim 1 wherein said host is a mammal.

3. The compound for use according to claim 1 wherein said host is a human.

4. The compound for use according to claim 1 wherein said host is a companion animal.

## Patentansprüche

1. Verbindung aus 1-(4-thio-[beta]-D-arabinofuranosyl)-Cytosin zur Verwendung in einem Verfahren zum Behandeln oder Verhüten von rheumatoider Arthritis, das bei einem Wirt, der dessen bedarf, ein Verabreichen einer Menge der Verbindung, die zum Behandeln oder Verhüten von rheumatoider Arthritis dient, aufweist.

2. Verbindung zur Verwendung nach Anspruch 1, wobei der Wirt ein Säugetier ist.

3. Verbindung zur Verwendung nach Anspruch 1, wobei der Wirt ein Mensch ist.

4. Verbindung zur Verwendung nach Anspruch 1, wobei der Wirt ein Haustier ist.

## Revendications

1. Composé consistant en 1-(4-thio-[béta]-D-arabinofuranosyl) cytosine pour une utilisation dans un procédé pour le traitement ou la prévention de la polyarthrite rhumatoïde qui comprend l'administration à un hôte qui le nécessite d'une quantité du composé efficace pour le traitement ou la prévention de la polyarthrite rhumatoïde.

2. Le composé pour une utilisation selon la revendication 1, ledit hôte étant un mammifère.

3. Le composé pour une utilisation selon la revendication 1, ledit hôte étant un être humain.

4. Le composé pour une utilisation selon la revendication 1, ledit hôte étant un animal de compagnie.
